# EUROPEAN PATENT APPLICATION

(11) **EP 4 066 840 A1**
(43) Date of publication of application: **05.10.2022**
(21) Application number: 20892583.4
(22) Date of filing: 26.11.2020
(51) Int. Cl.: A61K 31/538, A61P 1/16, A61P 3/04, A61P 35/00, A61P 43/00

(54) **PROPHYLACTIC OR THERAPEUTIC AGENT FOR NON-ALCOHOLIC STEATOHEPATITIS IN HUMANS**

(30) Priority: 26.11.2019 JP 2019213460
(71) Applicant: Mitsubishi Tanabe Pharma Corporation, Osaka-shi, Osaka 541-8505 (JP)
(72) Inventor: KIKKAWA, Kohei, Osaka-shi, Osaka 541-8505 (JP); OKA, Kozo, Osaka-shi, Osaka 541-8505 (JP)
(74) Representative: Hoffmann Eitle
(86) International application number: PCT/JP2020/044061
(87) International publication number: WO 2021/107029

(57) **Abstract**

The present invention aims to provide a prophylactic agent or therapeutic agent for human non-alcoholic steatohepatitis (NASH), the agent being capable of (i) reducing body weight, (ii) changing the amount of HA, the amount of PIIINP, and the amount of TIMP-1, in serum, and changing the amount of sVCAM-1 in plasma, or (iii) reducing liver stiffness. The object is achieved by a prophylactic agent or therapeutic agent for human non-alcoholic steatohepatitis (NASH), including: the 1,4-benzoxazine compound represented by Formula (I) or a pharmaceutically acceptable salt thereof as an effective component; and a pharmaceutically acceptable carrier; the agent being capable of (i) reducing body weight, (ii) changing the amount of HA, the amount of PIIINP, and the amount of TIMP-1, in serum, and changing the amount of sVCAM-1 in plasma, or (iii) reducing liver stiffness.

## Description

### TECHNICAL FIELD

The present invention relates to a prophylactic agent or therapeutic agent for human non-alcoholic steatohepatitis (NASH).

### BACKGROUND ART

The state in which a large amount of fat is accumulated in liver is known as fatty liver. Fatty liver can be roughly divided into alcoholic fatty liver and non-alcoholic fatty liver. A series of liver diseases ranging from non-alcoholic fatty liver to more advanced conditions such as steatohepatitis and liver cirrhosis are known as non-alcoholic fatty liver disease (NAFLD). Although most cases of NAFLD are non-alcoholic simple fatty liver, cases involving inflammation or fibrosis have the risk of progression to non-alcoholic steatohepatitis (NASH), non-alcoholic liver fibrosis, and liver cirrhosis, which may finally lead to development of hepatocellular carcinoma (HCC) (Patent Document 1, and Non-patent Documents 1 and 2).

The serum HA (hyaluronic acid) level has been described to be useful as a marker for prediction of the degree of liver fibrosis when it is combined with the serum autotaxin level (Patent Document 2).

On the other hand, a mineralocorticoid receptor regulator comprising the compound represented by the following Formula (I) is known (Patent Document 3). This document describes that the compound has affinity to the mineralocorticoid receptor, and that the compound is useful for prevention or treatment of various diseases with which the receptor is associated. Conventional mineralocorticoid receptor regulators not containing the compound are known to increase the body weight of subjects to whom the regulators are administered.

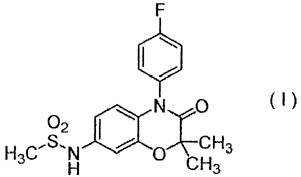

The compound represented by the Formula (I) is known to be useful for prevention or treatment of non-alcoholic steatohepatitis (NASH) (Patent Document 1). This document demonstrates the effectiveness of the compound represented by Formula (I) using non-alcoholic steatohepatitis (NASH) model rats as test animals, based on the results on the Sirius Red-positive area and the fibrosis score.

The compound represented by the Formula (I) is known to show crystal polymorphism, and to be effective for prevention or treatment of diseases caused by an increase in the mineralocorticoid receptor activity and/or an increase in the aldosterone level (Patent Document 4).

### PRIOR ART DOCUMENTS

### PATENT DOCUMENTS

[Patent Document 1] Japanese Translated PCT Patent Application Laid-open No. 2019-529477
[Patent Document 2] WO 2011/081214
[Patent Document 3] WO 2007/089034
[Patent Document 4] WO 2014/024950

### NON-PATENT DOCUMENTS

[Non-patent Document 1] D. A. Sass et al., Digestive Diseases and Sciences 2005; 50(1): 171-180
[Non-patent Document 2] J. K. Dyson et al., Frontline Gastroenterology, 2014; 5(3): 211-218

### SUMMARY OF THE INVENTION

### PROBLEMS TO BE SOLVED BY THE INVENTION

It has not been known that, in the prevention or treatment of human non-alcoholic steatohepatitis (NASH), the compound represented by the Formula (I) is capable of (i) reducing body weight, (ii) changing the amount of HA, the amount of PIIINP (N-Terminal Procollagen III Propeptide), and the amount of TIMP-1 (Tissue inhibitor for metalloproteinase-1), in serum, and changing the amount of sVCAM-1 (Soluble Vascular cell adhesion molecule) in plasma, or (iii) reducing liver stiffness.

An object of the present invention is to provide a prophylactic agent or therapeutic agent for human non-alcoholic steatohepatitis (NASH), the agent being capable of (i) reducing body weight, (ii) changing the amount of HA, the amount of PIIINP, and the amount of TIMP-1, in serum, and changing the amount of sVCAM-1 in plasma, or (iii) reducing liver stiffness.

### MEANS FOR SOLVING THE PROBLEMS

As a result of intensive study under such circumstances, the present inventors discovered that the above problem can be solved by a prophylactic agent or therapeutic agent for human non-alcoholic steatohepatitis (NASH), comprising:
the 1,4-benzoxazine compound represented by the following Formula (I): 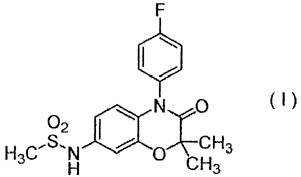 or a pharmaceutically acceptable salt thereof; and
a pharmaceutically acceptable carrier; thereby completing the present invention.

More specifically, the present invention is as follows.
<1> A prophylactic agent or therapeutic agent for human non-alcoholic steatohepatitis (NASH), comprising:
   the 1,4-benzoxazine compound represented by the following Formula (I): 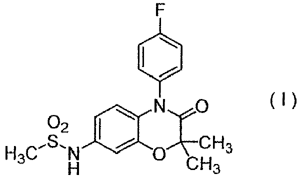 or a pharmaceutically acceptable salt thereof, as an effective component; and
   a pharmaceutically acceptable carrier;
   the agent being capable of reducing body weight.
<2> A prophylactic agent or therapeutic agent for human non-alcoholic steatohepatitis (NASH), comprising:
   the 1,4-benzoxazine compound represented by the following Formula (I): 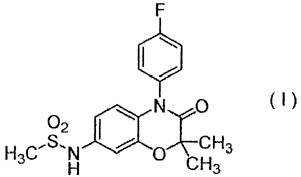 or a pharmaceutically acceptable salt thereof, as an effective component; and
   a pharmaceutically acceptable carrier; the agent being capable of changing the amount of HA, the amount of PIIINP, and the amount of TIMP-1, in serum, and changing the amount of sVCAM-1 in plasma.
<3> A prophylactic agent or therapeutic agent for human non-alcoholic steatohepatitis (NASH), comprising:
   the 1,4-benzoxazine compound represented by the following Formula (I): 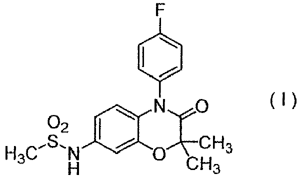 or a pharmaceutically acceptable salt thereof, as an effective component; and
   a pharmaceutically acceptable carrier;
   the agent being capable of reducing liver stiffness.
<4> The prophylactic agent according to any one of <1> to <3>, capable of suppressing progression from non-alcoholic simple fatty liver to non-alcoholic steatohepatitis (NASH).
<5> The prophylactic agent or therapeutic agent according to any one of <1> to <3>, capable of suppressing progression from non-alcoholic steatohepatitis (NASH) to non-alcoholic liver fibrosis.
<6> The prophylactic agent or therapeutic agent according to any one of <1> to <3>, capable of suppressing progression from non-alcoholic steatohepatitis (NASH) to liver cancer.
<7> A method of prevention or treatment of human non-alcoholic steatohepatitis (NASH), comprising:
   administering a prophylactically or therapeutically effective amount of the 1,4-benzoxazine compound represented by the following Formula (I): 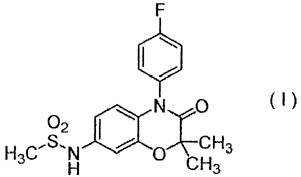 or a pharmaceutically acceptable salt thereof to a patient in need of the prevention or treatment;
   the method being capable of reducing body weight.
<8> A method of prevention or treatment of human non-alcoholic steatohepatitis (NASH), comprising:
   administering a prophylactically or therapeutically effective amount of the 1,4-benzoxazine compound represented by the following Formula (I): 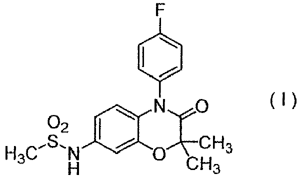 or a pharmaceutically acceptable salt thereof to a patient in need of the prevention or treatment;
   the method being capable of changing the amount of HA, the amount of PIIINP, and the amount of TIMP-1, in serum, and changing the amount of sVCAM-1 in plasma.
<9> A method of prevention or treatment of human non-alcoholic steatohepatitis (NASH), comprising:
   administering a prophylactically or therapeutically effective amount of the 1,4-benzoxazine compound represented by the following Formula (I): 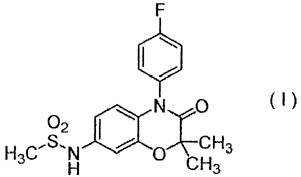 or a pharmaceutically acceptable salt thereof to a patient in need of the prevention or treatment;
   the method being capable of reducing liver stiffness.
<10> Use of the 1,4-benzoxazine compound represented by the following Formula (I): 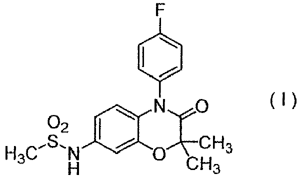 or a pharmaceutically acceptable salt thereof, for the production of a prophylactic agent or therapeutic agent for human non-alcoholic steatohepatitis (NASH), the agent being capable of reducing body weight.
<11> Use of the 1,4-benzoxazine compound represented by the following Formula (I): 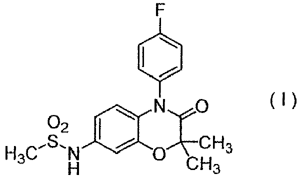 or a pharmaceutically acceptable salt thereof, for the production of a prophylactic agent or therapeutic agent for human non-alcoholic steatohepatitis (NASH), the agent being capable of changing the amount of HA, the amount of PIIINP, and the amount of TIMP-1, in serum, and changing the amount of sVCAM-1 in plasma.
<12> Use of the 1,4-benzoxazine compound represented by the following Formula (I): 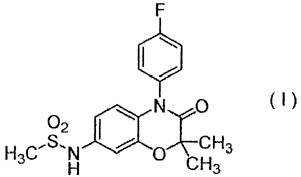 or a pharmaceutically acceptable salt thereof, for the production of a prophylactic agent or therapeutic agent for human non-alcoholic steatohepatitis (NASH), the agent being capable of reducing liver stiffness.

### EFFECT OF THE INVENTION

The present invention can provide a pharmaceutical which is effective for prevention or treatment of human non-alcoholic steatohepatitis (NASH), and which can be safely administered, the pharmaceutical being capable of (i) reducing body weight, (ii) changing the amount of HA, the amount of PIIINP, and the amount of TIMP-1, in serum, and changing the amount of sVCAM-1 in plasma, or (iii) reducing liver stiffness.

### BRIEF DESCRIPTION OF THE DRAWINGS

Fig. 1 is a graph showing the average amount of change in the body weight after the assignment in Examples.
Fig. 2 is a graph showing the average amount of change in the serum hyaluronic acid level after the assignment in Examples.
Fig. 3-1 is a graph showing the average amount of change in the serum PIIINP level after the assignment in Examples.
Fig. 3-2 is a graph showing the average amount of change in the serum PIIINP level after the assignment in Examples.
Fig. 3-3 is a graph showing the average amount of change in the serum PIIINP level after the assignment in Examples.

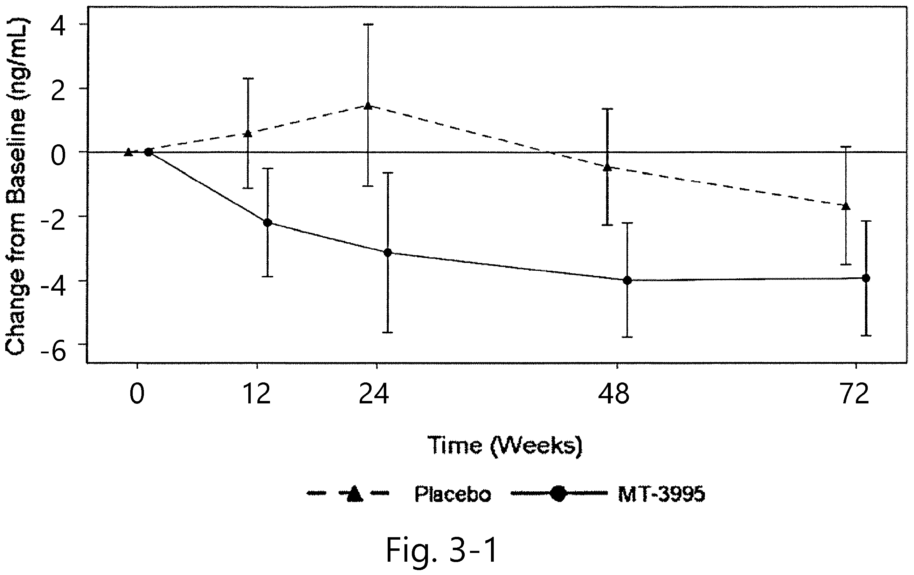

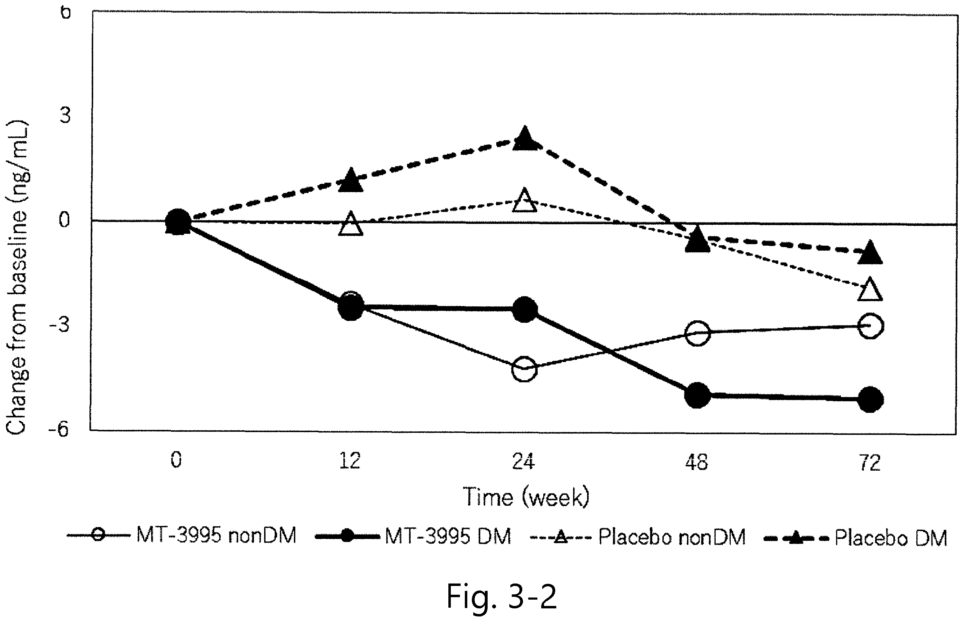

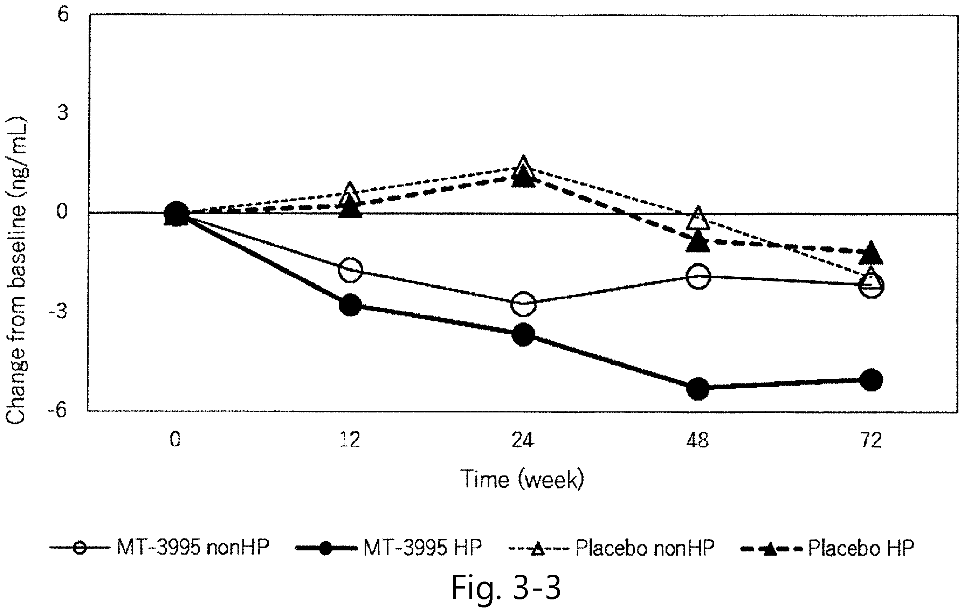

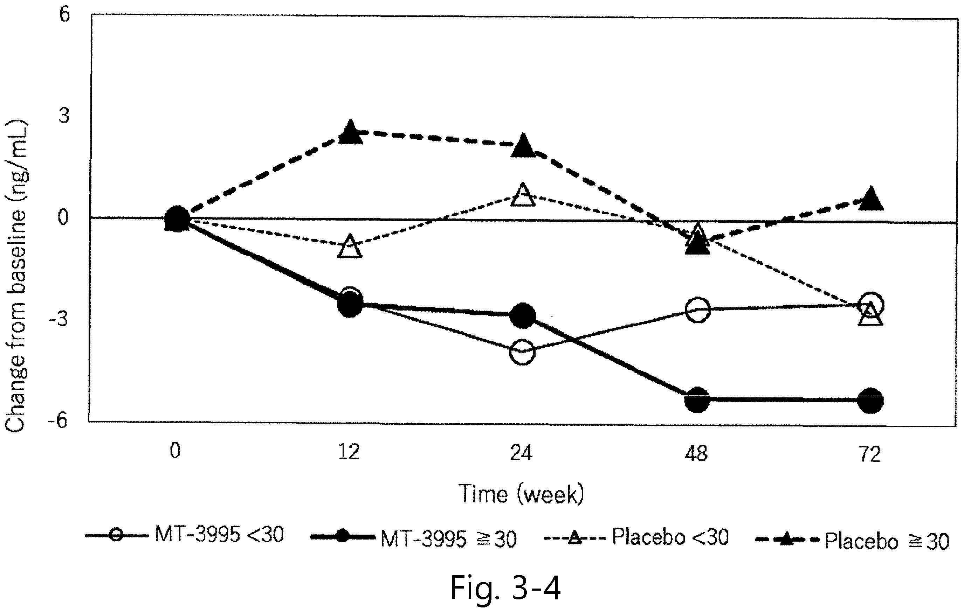

Fig. 3-4 is a graph showing the average amount of change in the serum PIIINP level after the assignment in Examples.

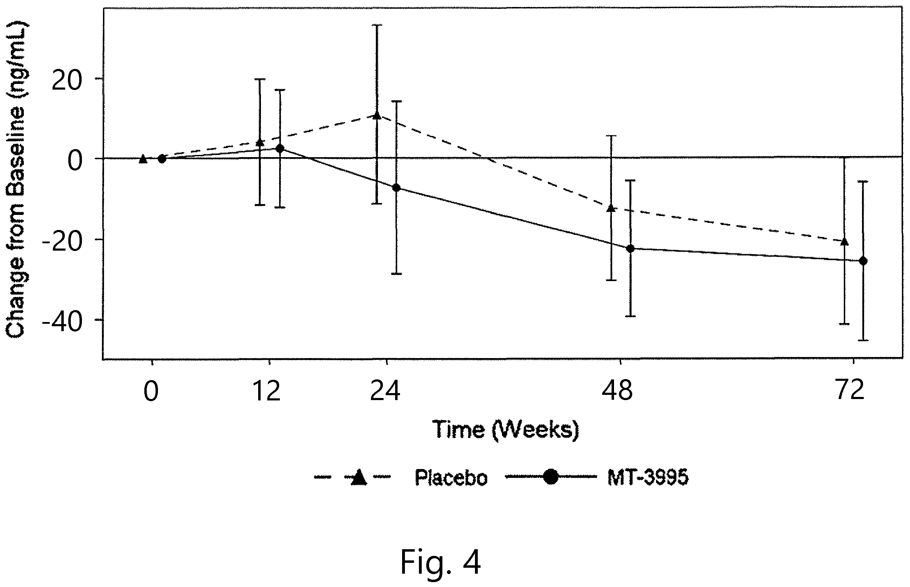

Fig. 4 is a graph showing the average amount of change in the serum TIMP-1 level after the assignment in Examples.

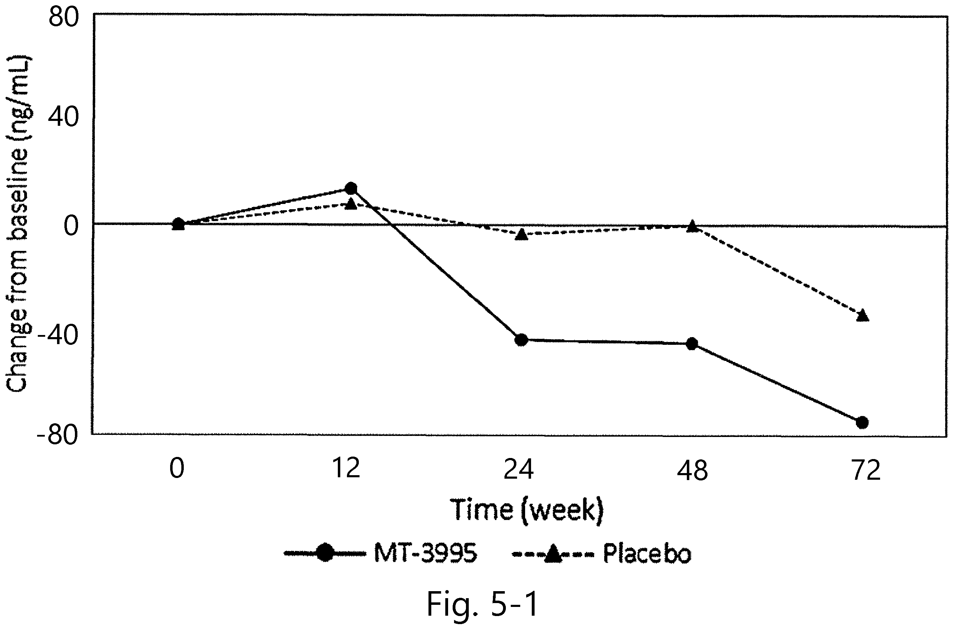

Fig. 5-1 is a graph showing the average amount of change in the plasma sVCAM-1 level after the assignment in Examples.

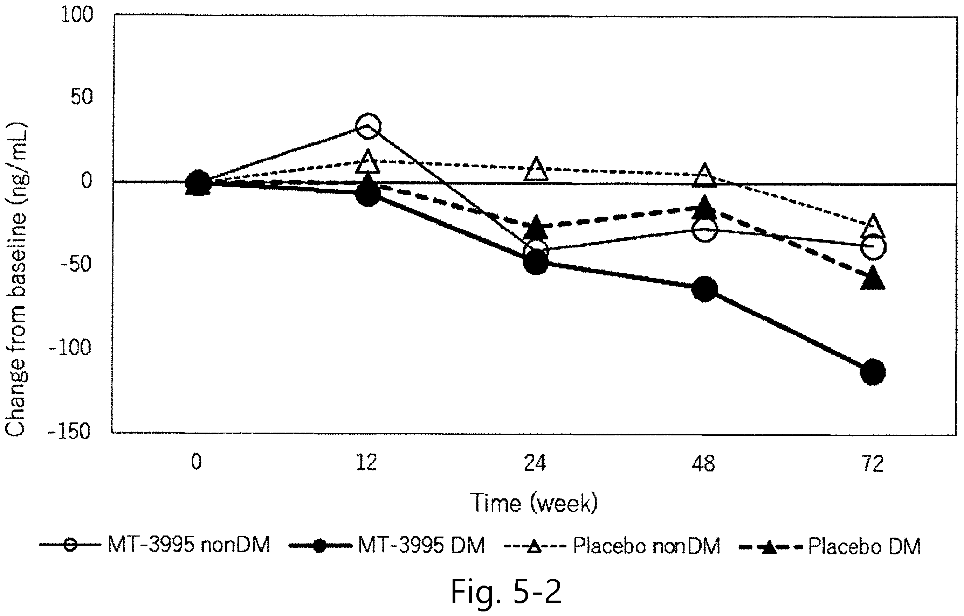

Fig. 5-2 is a graph showing the average amount of change in the plasma sVCAM-1 level after the assignment in Examples.

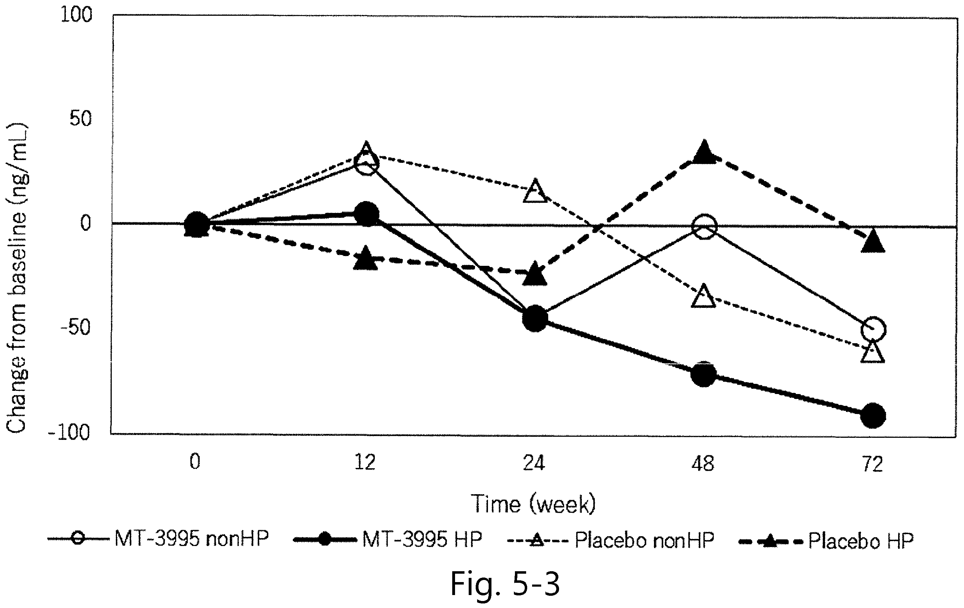

Fig. 5-3 is a graph showing the average amount of change in the plasma sVCAM-1 level after the assignment in Examples.

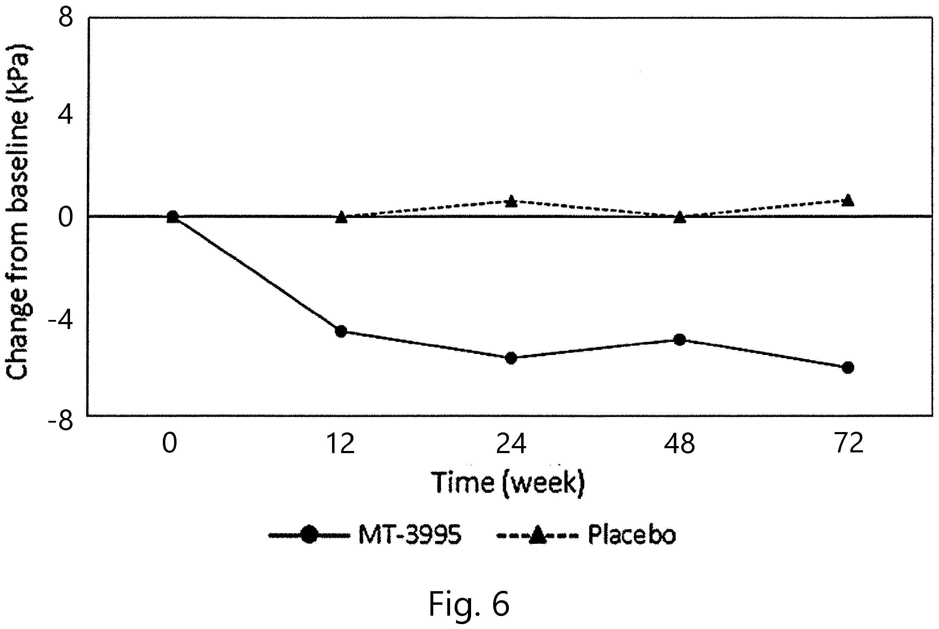

Fig. 6 is a graph showing the average amount of change in the liver stiffness after the assignment, based on the measurement using Shear Wave Elastography in Examples.

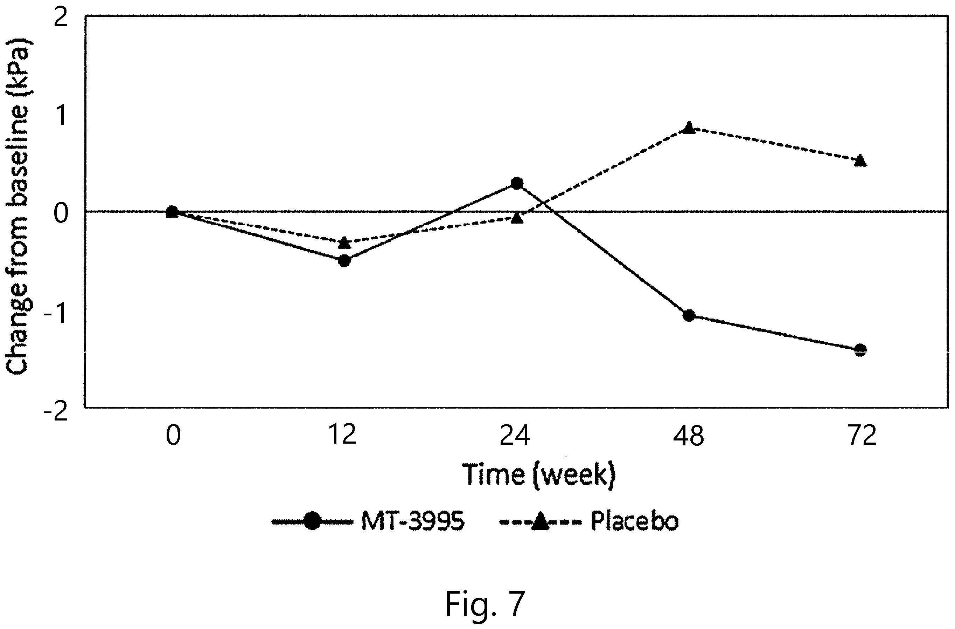

Fig. 7 is a graph showing the average amount of change in the liver stiffness after the assignment, based on the measurement using Fibro scan in Examples.

### MODE FOR CARRYING OUT THE INVENTION

The present invention is described below in detail.

In the present description, any "fiber/fibre" generated in a living body is referred to as "fiber".

The term "non-alcoholic simple fatty liver" used in the present description means a disease showing only fat deposition in hepatocytes.

The term "non-alcoholic steatohepatitis (NASH)" used in the present description means a disease showing not only fat deposition, but also findings similar to those of alcoholic hepatitis (such as inflammation, hepatic necrosis, ballooning degeneration, and/or fibrosis).

The term "non-alcoholic liver fibrosis" used in the present description means a disease showing not only advanced fibrosis in the liver tissue, but also excessive production and/or accumulation of collagen and/or another/other component(s) of the extracellular matrix.

The term "non-alcoholic liver cirrhosis" used in the present description means a disease in which the hepatic lobule structure is modified due to advanced fibrosis.

One aspect of the present invention relates to a prophylactic agent or therapeutic agent for human non-alcoholic steatohepatitis (NASH), comprising:
the 1,4-benzoxazine compound represented by the following Formula (I): 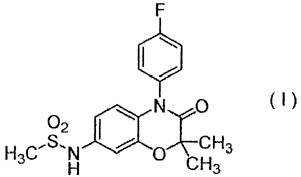 or a pharmaceutically acceptable salt thereof, as an effective component; and
a pharmaceutically acceptable carrier;
the agent being capable of reducing body weight.

The 1,4-benzoxazine compound represented by the Formula (I) (which may be referred to as "Compound (I) in the present description) contained as an effective component in the prophylactic agent or therapeutic agent of the present invention is a known compound described in Patent Document 3. Compound (I) may be used for a pharmaceutical use either in the free form, or in the form of a pharmaceutically acceptable salt thereof.

Examples of the pharmaceutically acceptable salt include salts with an acid, such as inorganic acid salts (including hydrochloric acid salt, sulfuric acid salt, phosphoric acid salt, and hydrobromic acid salt), and organic acid salts (including acetic acid salt, fumaric acid salt, oxalic acid salt, citric acid salt, methanesulfonic acid salt, benzenesulfonic acid salt, tosic acid salt, and maleic acid salt). Other examples of the pharmaceutically acceptable salt include salts with a base, such as alkali metal salts (including sodium salt and potassium salt) and alkaline earth metal salts (including calcium salt).

Compound (I) or the pharmaceutically acceptable salt thereof includes any of the inner salts and adducts thereof, and the solvates and hydrates thereof.

Compound (I) is known to show crystal polymorphism (Patent Document 4). The Compound (I) used for the prophylactic agent or therapeutic agent of the present invention may be any of the crystal polymorphs.

The prophylactic agent or therapeutic agent of the present invention may be orally or parenterally administered to a human. Examples of the parenteral administration include intravenous administration, intramuscular administration, subcutaneous administration, intraorgan administration, intranasal administration, intradermal administration, ocular instillation, intracerebral administration, rectal administration, vaginal administration, intraperitoneal administration, and intralesional administration.

The dosage form of the prophylactic agent or therapeutic agent of the present invention is not limited. Examples of the dosage form include tablets, pills, granules, capsules, powders, injection solutions, inhalants, solutions, emulsions, and suppositories.

In cases of a solid preparation, examples of the pharmaceutically acceptable carrier that may be used include excipients, lubricants, binders, and disintegrators. In cases of a liquid preparation, examples of the carrier that may be used include solvents, solubilizers, suspending agents, isotonic agents, and buffers. When necessary, other additives (for example, an antiseptic) may also be added.

Specific examples of the carrier include binders such as gelatin, corn starch, tragacanth gum, and gum arabic; excipients such as starch and crystalline cellulose; swelling agents such as alginic acid; injection solvents such as water, ethanol, and glycerin; and adhesives such as rubber adhesives and silicone adhesives.

The prophylactic agent or therapeutic agent of the present invention can be expected to show only low toxicity and low side effects, and is an excellent pharmaceutical. Thus, the prophylactic agent or therapeutic agent of the present invention can be safely administered to a human. The human is preferably a human in need of prevention or treatment of non-alcoholic steatohepatitis (NASH).

The human preferably has, but does not necessarily need to have, a Body Mass Index (which may be referred to as "BMI") of not less than 30. It is well known that BMI can be calculated by measuring the body height and the body weight by known methods.

The human is preferably a human complicated with another/other disease(s) and/or the like (including diseases, disorders, symptoms, and signs, in the present description). Examples of the other disease(s) include diabetes, hyperglycemia, hypertension, obesity, glucose intolerance, insulin resistance, metabolic syndrome, and dyslipidemia (hyperlipidemia). The other disease(s) is/are preferably diabetes and/or hypertension.

Compound (I) or the pharmaceutically acceptable salt thereof as an effective component of the prophylactic agent or therapeutic agent of the present invention is effective for prevention or treatment of human non-alcoholic steatohepatitis (NASH), and has an action that reduces the body weight of the human.

The "reduction of the body weight" means that administration of Compound (I) or the pharmaceutically acceptable salt thereof to a human results in a lower body weight of the human relative to that in a case where it is not administered. The human body weight can be measured with a body weight scale or the like.

In the present description, the "treatment" includes curing of a disease or the like (such as the entire pathological conditions, or one or more pathological conditions), improvement of the disease or the like, and suppression of an increase in the severity of the disease or the like. The "therapeutically effective amount" means a dose of the therapeutic agent of the present invention, which dose is sufficient for achieving such an object.

The "prevention" in the present description includes prevention of development of a disease or the like, and slowing of development of the disease or the like. The "prophylactically effective amount" means a dose of the prophylactic agent of the present invention, which dose is sufficient for achieving such an object.

The total amount of Compound (I) or the pharmaceutically acceptable salt thereof in the total amount of the prophylactic agent or therapeutic agent of the present invention may be appropriately set depending on the dosage form, administration method, carrier, and/or the like. It is usually not less than 0.01% (w/w), preferably not less than 0.05% (w/w), more preferably not less than 0.1% (w/w), but is usually not more than 99% (w/w), preferably not more than 90% (w/w), more preferably not more than 85% (w/w).

The dose of the prophylactic agent or therapeutic agent of the present invention is appropriately set depending on the subject to whom the agent is to be administered, the administration route, the age of the subject, the sex of the subject, symptoms, severity of the symptoms, the dosage form, the usage, and/or the like. The dose is not limited as long as the prophylactic effect or therapeutic effect on non-alcoholic steatohepatitis (NASH) can be produced in the subject to whom the agent is administered. In any case, the agent may be administered once daily, or dividedly a plurality of times daily. In a specific example for oral administration to an adult patient (with a body weight of about 40 kg to 80 kg, for example, 60 kg), the daily dose in terms of the total amount of Compound (I) or the pharmaceutically acceptable salt thereof is usually not less than 0.1 mg, preferably not less than 0.5 mg, more preferably not less than 1 mg, but is usually not more than 50 mg, preferably not more than 40 mg, more preferably not more than 30 mg.

The dosing period of the prophylactic agent or therapeutic agent of the present invention is appropriately set depending on the subject to whom the agent is to be administered, the administration route, the age of the subject, the sex of the subject, symptoms, severity of the symptoms, the dosage form, the usage, and/or the like. The dosing period is not limited as long as the prophylactic effect or therapeutic effect on non-alcoholic steatohepatitis (NASH) can be produced in the subject to whom the agent is administered. In a specific example for oral administration to an adult patient (with a body weight of about 40 kg to 80 kg, for example, 60 kg), the dosing period regarding its lower limit is, for example, not less than 6 weeks, not less than 12 weeks, not less than 18 weeks, not less than 24 weeks, not less than 30 weeks, not less than 36 weeks, not less than 42 weeks, not less than 48 weeks, not less than 54 weeks, not less than 60 weeks, not less than 66 weeks, or not less than 72 weeks. Regarding the upper limit of the dosing period, the agent may be administered for the period used for prevention or treatment (including prognosis) of non-alcoholic steatohepatitis (NASH). Examples of the dosing period include, but are not limited to, the period until death, and a period of not more than 50 years, not more than 25 years, not more than 10 years, not more than 5 years, not more than 3 years, not more than 1.5 years, or not more than 72 weeks.

The prophylactic agent or therapeutic agent of the present invention can be produced by a known method. The prophylactic agent or therapeutic agent of the present invention may be molded into a sustained release formulation comprising the effective component.

The prophylactic agent or therapeutic agent of the present invention may be a prophylactic agent capable of suppressing progression from non-alcoholic simple fatty liver to non-alcoholic steatohepatitis.

The prophylactic agent or therapeutic agent of the present invention may be a prophylactic agent or therapeutic agent capable of suppressing progression from non-alcoholic steatohepatitis to non-alcoholic liver fibrosis.

The prophylactic agent or therapeutic agent of the present invention may be a prophylactic agent or therapeutic agent capable of suppressing progression from non-alcoholic steatohepatitis to liver cancer.

Another aspect of the present invention relates to a prophylactic agent or therapeutic agent for human non-alcoholic steatohepatitis (NASH), comprising:
the 1,4-benzoxazine compound represented by the following Formula (I): 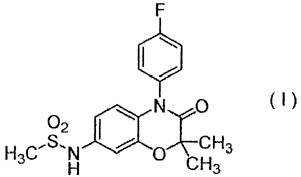 or a pharmaceutically acceptable salt thereof, as an effective component; and
a pharmaceutically acceptable carrier;
the agent being capable of changing the amount of HA, the amount of PIIINP, and the amount of TIMP-1, in serum, and changing the amount of sVCAM-1 in plasma.

Compound (I) or the pharmaceutically acceptable salt thereof as an effective component of the prophylactic agent or therapeutic agent of the present invention is effective for prevention or treatment of human non-alcoholic steatohepatitis (NASH), and has an action that changes one or more selected from the group consisting of the amount of HA, the amount of PIIINP, and the amount of TIMP-1, in serum, and the amount of sVCAM-1 in plasma, of the human.

The prophylactic agent or therapeutic agent of the present invention preferably has an action that changes the amount of HA, the amount of PIIINP, and the amount of TIMP-1, in serum, and the amount of sVCAM-1 in plasma.

Each change is an increase or a decrease, preferably a decrease.

The term "increasing the amount of HA in serum" means that administration of Compound (I) or the pharmaceutically acceptable salt thereof to a human results in a larger amount of HA in serum of the human relative to that in a case where it is not administered.

The term "decreasing the amount of HA in serum" means that administration of Compound (I) or the pharmaceutically acceptable salt thereof to a human results in a smaller amount of HA in serum of the human relative to that in a case where it is not administered.

The amount of HA in serum of a human can be measured by, for example, preparing serum from blood collected from the human according to a conventional method, and using a commercially available kit, as described later in Examples.

The definitions and the measurement method described above similarly apply to the terms "changing the amount of PIIINP in serum", "changing the amount of TIMP-1 in serum", and "changing the amount of sVCAM-1 in plasma".

To details of the present aspect, the above description on the prophylactic agent or therapeutic agent for human non-alcoholic steatohepatitis (NASH), comprising: the 1,4-benzoxazine compound represented by the above Formula (I) or a pharmaceutically acceptable salt thereof, as an effective component; and a pharmaceutically acceptable carrier; the agent being capable of reducing body weight, is applied.

Another aspect of the present invention relates to a prophylactic agent or therapeutic agent for human non-alcoholic steatohepatitis (NASH), comprising:
the 1,4-benzoxazine compound represented by the following Formula (I): 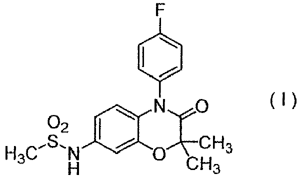 or a pharmaceutically acceptable salt thereof, as an effective component; and
a pharmaceutically acceptable carrier;
the agent being capable of reducing liver stiffness.

Compound (I) or the pharmaceutically acceptable salt thereof as an effective component of the prophylactic agent or therapeutic agent of the present invention is effective for prevention or treatment of human non-alcoholic steatohepatitis (NASH), and has an action that reduces the liver stiffness of the human.

The "reduction of the liver stiffness" means that administration of Compound (I) or the pharmaceutically acceptable salt thereof to a human results in a lower liver stiffness of the human relative to that in a case where it is not administered.

The liver stiffness of a human can be measured using an apparatus such as Shear Wave Elastography or Fibro scan as described later in Examples.

To details of the present aspect, the above description on the prophylactic agent or therapeutic agent for human non-alcoholic steatohepatitis (NASH), comprising: the 1,4-benzoxazine compound represented by the above Formula (I) or a pharmaceutically acceptable salt thereof, as an effective component; and a pharmaceutically acceptable carrier; the agent being capable of reducing body weight, is applied.

### EXAMPLES

The present invention is described below in more detail according to Examples. However, the scope of the present invention is not limited by these Examples. MT-3995, which was used in the following Examples, means the Compound (I).

### [Test Protocol]

- Number of cases to be analyzed: 47 cases (placebo administration group: 23 cases; MT-3995 administration group: 24 cases)
- The subjects were selected according to the following criteria.
   NAFLD activity score ≥ 4
   Kleiner fibrosis classification: Stage 2 or Stage 3
   ALT at 10 weeks before assignment and 2 weeks before assignment ≥ 60
- Patient backgrounds are shown in Table 1.

### [Table 1]

**Table 1. Patient backgrounds**

| | Placebo | MT-3995 | Total |
|---|---|---|---|
| Number of subjects^{*1} | 23 | 24 | 47 |
| Sex (male)^{*1} | 13 (56.5) | 12 (50.0) | 25 (53.2) |
| Age (years old)^{*2} | 53.0 (31 - 75) | 52.0 (40 - 67) | 53.0 (31 - 75) |
| Fifty years old or older^{*1} | 13 (56.5) | 14 (58.3) | 27 (57.4) |
| Body weight (kg)^{*2} | 76.70 (51.8 - 100.5) | 81.10 (45.6 - 108.3) | 78.20 (45.6 - 108.3) |
| BMI (kg/m²)^{*2} | 29.31 (21.8 - 36.3) | 30.21 (21.7 - 39.8) | 29.73 (21.7 - 39.8) |
| Complication^{*1} | | | |
| Diabetes | 8 (34.8) | 12 (50.0) | 20 (42.6) |
| Dyslipidemia | 13 (56.5) | 15 (62.5) | 28 (59.6) |
| Hypertension | 12 (52.2) | 16 (66.7) | 28 (59.6) |
| SBP (mmHg)^{*2} | 130.0 (115 - 159) | 136.0 (110 - 155) | 130.0 (110 - 159) |
| DBP (mmHg)^{*2} | 84.0 (66 - 98) | 85.5 (65 - 98) | 85.0 (65 - 98) |
| ALT (U/L) | 90.0 (64 - 168) | 102.5 (49 - 174) | 93.0 (49 - 174) |
| ALT (U/L) > 90^{*1} | 11 (47.8) | 14 (58.3) | 25 (53.2) |

| | | | |
|---|---|---|---|
| *1: Number of subjects (% ratio) *2: Median (Minimum value - Maximum value) | | | |

- Pre-observation period (number of weeks): 10 weeks
- Administration period (number of weeks): 72 weeks
- Post-observation period (number of weeks): 8 weeks
- Dosage and administration: MT-3995 administration group = once daily, 10 mg; placebo group = once-daily administration

### (Example 1: Body Weight Change)

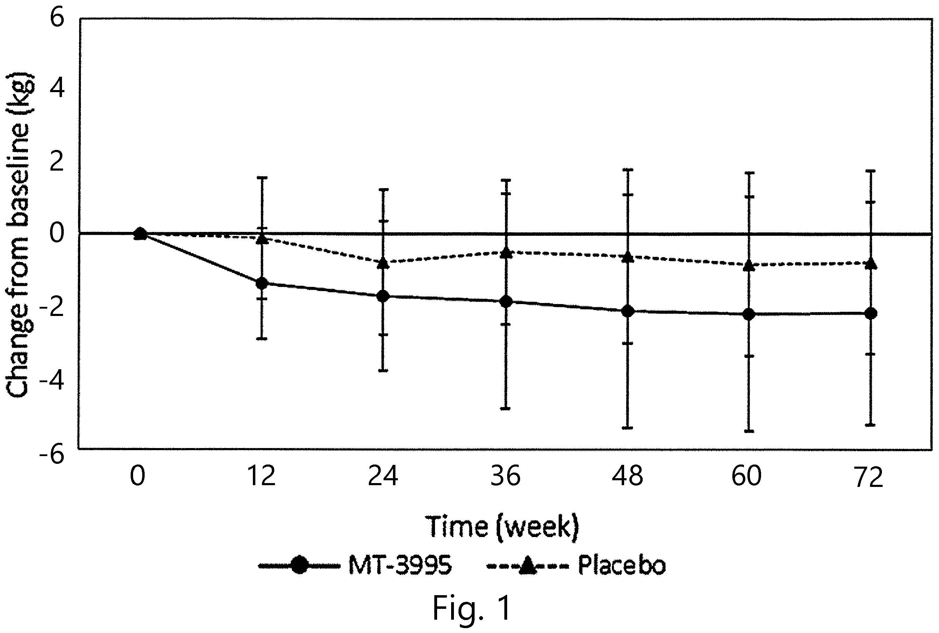

Table 2 and Fig. 1 show the average amount of change in the body weight after the assignment, in the placebo group and the MT-3995 group. The body weight was measured at the time of the visit. A decrease in the body weight was found from the beginning of the administration in the MT-3995 administration group, and from Week 12 of the administration in the placebo group. The rate of body weight reduction was higher in the MT-3995 administration group than in the placebo group.

### [Table 2]

**Table 2. Average amount of change in the body weight after the assignment**

| | Change from baseline / Weight (kg) | | | |
|---|---|---|---|---|
| | Placebo | | MT-3995 | |
| Visit | Mean (SD) | (n) | Mean (SD) | (n) |
| Week 12 | 0.13 (1.68) | 23 | -1.39 (1.55) | 24 |
| Week 24 | -0.80 (2.02) | 23 | -1.73 (2.09) | 24 |
| Week 36 | -0.51 (2.01) | 22 | -1.88 (2.99) | 22 |
| Week 48 | -0.63 (2.43) | 21 | -2.15 (3.23) | 22 |
| Week 60 | -0.86 (2.55) | 21 | -2.23 (3.25) | 22 |
| Week 72 | -0.78 (2.55) | 21 | -2.21 (3.10) | 22 |

### (Example 2: Change in Serum Hyaluronic Acid Level)

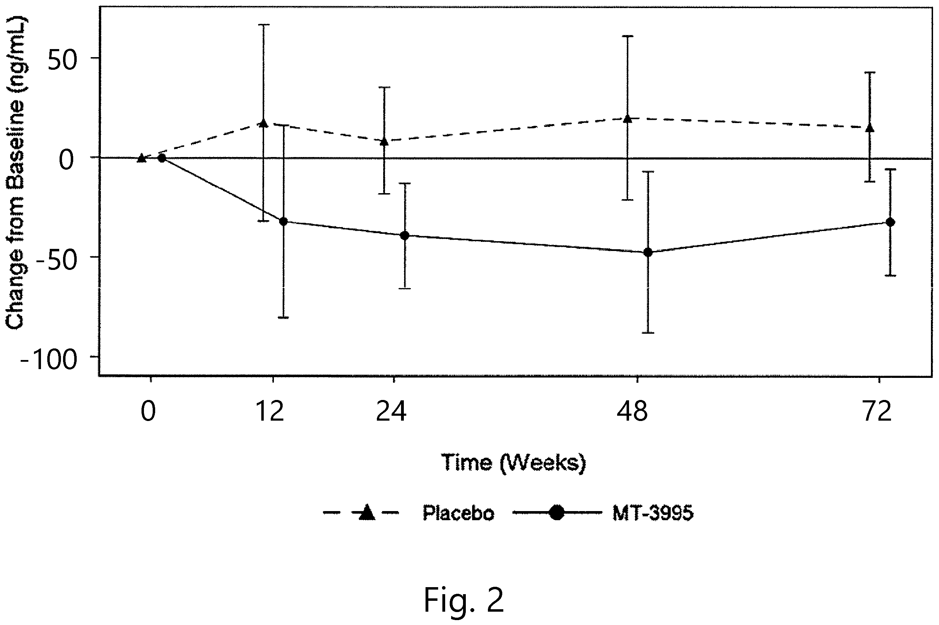

Table 3 and Fig. 2 show the average amount of change in the serum hyaluronic acid level after the assignment. For measurement of the serum hyaluronic acid level, serum was prepared from blood collected from each patient on the day of the visit, according to a conventional method. The prepared serum was subjected to a test. For the measurement, Chemilumi Hyaluronic Acid (Siemens Healthcare Diagnostics K. K.) was used.

Throughout the treatment period, the serum hyaluronic acid level in the placebo group was higher than the level observed at the beginning of the administration. On the other hand, throughout the treatment period, the serum hyaluronic acid level in the MT-3995 group was lower than the level observed at the beginning of the administration. At Weeks 24, 48, and 72 after the beginning of the administration, the serum hyaluronic acid level in the MT-3995 administration group was significantly lower than the level in the placebo group.

### [Table 3]

**Table 3. Average amount of change in the serum hyaluronic acid level after the assignment**

| | Change from baseline / Hyaluronic Acid (ng/mL) | | | |
|---|---|---|---|---|
| | Placebo | | MT-3995 | |
| Visit | LS Mean (95 % Cl) | (n) | LS Mean (95 % CI) | (n) |
| Week 12 | 17.65 (-31.59, 66.89) | 23 | -31.87 (-80.13, 16.39) | 24 |
| Week 24 | 8.78 (-18.00, 35.56) | 23 | -39.02 (-65.34, -12.69) | 24 |
| Week 48 | 20.06 (-21.08, 61.21) | 21 | -47.23 (-87.58, -6.87) | 22 |
| Week 72 | 15.67 (-11.65, 42.99) | 21 | -32.02 (-58.74, -5.31) | 22 |

### (Example 3: Change in Serum PIIINP Level)

Table 4-1 and Fig. 3-1 show the average amount of change in the serum procollagen III N-Terminal Propeptide (PIIINP) level after the assignment. For measurement of the serum PIIINP level, serum was prepared from blood collected from each patient, according to a conventional method. The prepared serum was subjected to a test. For the measurement, Chemilumi PIIIP (Siemens Healthcare Diagnostics K. K.) was used.

Throughout the treatment period, the serum PIIINP value in the placebo group did not largely change from the value observed at the beginning of the administration. However, throughout the treatment period, the serum PIIINP value in the MT-3995 group was lower than the value observed at the beginning of the administration. At Weeks 12, 24, and 48 after the beginning of the administration, the serum PIIINP level in the MT-3995 administration group was significantly lower than the level in the placebo group.

### [Table 4-1]

**Table 4-1. Average amount of change in the serum PIIINP level after the assignment**

| | Change from baseline / PIIINP (ng/mL) | | | |
|---|---|---|---|---|
| | Placebo | | MT-3995 | |
| Visit | LS Mean (95 % CI) | (n) | LS Mean (95 % CI) | (n) |
| Week 12 | 0.591 (-1.121, 2.303) | 23 | -2.192 (-3.874, -0.510) | 24 |
| Week 24 | 1.469 (-1.062, 3.999) | 23 | -3.124 (-5.605, -0.643) | 24 |
| Week 48 | -0.457 (-2.270, 1.356) | 21 | -3.987 (-5.756, -2.218) | 22 |
| Week 72 | -1.664 (-3.489, 0.161) | 21 | -3.930 (-5.714, -2.146) | 22 |

A stratified analysis was carried out for the subjects based on the presence or absence of complication with diabetes. The results are shown in Table 4-2 and Fig. 3-2. DM represents the "group of patients complicated with diabetes". A higher effect was found in the group of patients complicated with diabetes.

### [Table 4-2]

**Table 4-2. Average amount of change in the serum PIIINP level after the assignment**

| | Change from baseline / PIIINP (ng/mL) | | | | | | | |
|---|---|---|---|---|---|---|---|---|
| | Placebo | | | | MT-3995 | | | |
| | non-DM | | DM | | non-DM | | DM | |
| Visit | Mean | (n) | Mean | (n) | Mean | (n) | Mean | (n) |
| Week 12 | -0.03 | 15 | 1.23 | 8 | -2.34 | 12 | -2.45 | 12 |
| Week 24 | 0.67 | 15 | 2.45 | 8 | -4.19 | 12 | -2.47 | 12 |
| Week 48 | -0.45 | 15 | -0.40 | 8 | -3.14 | 12 | -4.92 | 12 |
| Week 72 | -1.84 | 15 | -0.79 | 8 | -2.89 | 12 | -5.00 | 12 |

Further, a stratified analysis was carried out for the subjects based on the presence or absence of complication with hypertension. The results are shown in Table 4-3 and Fig. 3-3. HP represents the "group of patients complicated with hypertension". A higher effect was found in the group of patients complicated with hypertension.

### [Table 4-3]

**Table 4-3. Average amount of change in the serum PIIINP level after the assignment**

| | Change from baseline / PIIINP (ng/mL) | | | | | | | |
|---|---|---|---|---|---|---|---|---|
| | Placebo | | | | MT-3995 | | | |
| | non-HP | | HP | | non-HP | | HP | |
| Visit | Mean | (n) | Mean | (n) | Mean | (n) | Mean | (n) |
| Week 12 | 0.61 | 11 | 0.23 | 12 | -1.69 | 8 | -2.75 | 16 |
| Week 24 | 1.42 | 11 | 1.16 | 12 | -2.71 | 8 | -3.64 | 16 |
| Week 48 | -0.11 | 11 | -0.79 | 12 | -1.89 | 8 | -5.26 | 16 |
| Week 72 | -1.89 | 11 | -1.15 | 12 | -2.13 | 8 | -4.98 | 16 |

Further, an analysis was carried out after dividing the subjects into patients with a BMI of not less than 30 and patients with a BMI of less than 30. The results are shown in Table 4-4 and Fig. 3-4. In the figure, "<30" represents the group of patients with a BMI of less than 30, and "≥30" represents the group of patients with a BMI of not less than 30. A higher effect was found in the group of patients with a BMI of not less than 30.

### [Table 4-4]

**Table 4-4. Average amount of change in the serum PIIINP level after the assignment**

| | Change from baseline / PIIINP (ng/mL) | | | | | | | |
|---|---|---|---|---|---|---|---|---|
| | Placebo | | | | MT-3995 | | | |
| | BMI < 30 | | BMI ≧ 30 | | BMI < 30 | | BMI ≧ 30 | |
| Visit | Mean | (n) | Mean | (n) | Mean | (n) | Mean | (n) |
| Week 12 | -0.76 | 15 | 2.61 | 8 | -2.33 | 12 | -2.46 | 12 |
| Week 24 | 0.78 | 15 | 2.25 | 8 | -3.86 | 12 | -2.79 | 12 |
| Week 48 | -0.35 | 15 | -0.61 | 8 | -2.58 | 12 | -5.24 | 12 |
| Week 72 | -2.67 | 15 | 0.72 | 8 | -2.40 | 12 | -5.24 | 12 |

### (Example 4: Change in Serum TIMP-1 Level)

Table 5 and Fig. 4 show the average amount of change in the serum TIMP-1 level after the assignment. For measurement of the serum TIMP-1 level, serum was prepared from blood collected from each patient, according to a conventional method. The prepared serum was subjected to a test. For the measurement, ADVIA Centaur (registered trademark) TIMP-1 assay (Siemens Healthcare Diagnostics K. K.) was used.

The placebo group showed serum TIMP-1 values higher than the value observed at the beginning of the administration, until Week 24 after the beginning of the treatment. The TIMP-1 value decreased thereafter. However, the MT-3995 administration group showed serum TIMP-1 values lower than the value observed at the beginning of the administration, from Week 24 after the beginning of the treatment. Throughout the period of the clinical trial, this group showed values lower than the values in the Placebo group.

### [Table 5]

**Table 5. Average amount of change in the serum TIMP-1 level after the assignment**

| | Change from baseline / TIMP-1 (ng/mL) | | | |
|---|---|---|---|---|
| | Placebo | | MT-3995 | |
| Visit | LS Mean (95 % Cl) | (n) | LS Mean (95 % Cl) | (n) |
| Week 12 | 4.1 (-11.6, 19.8) | 23 | 2.4 (-12.3, 17.1) | 24 |
| Week 24 | 11.0 (-11.3, 33.3) | 23 | -7.2 (-28.6, 14.2) | 24 |
| Week 48 | -12.3 (-30.2, 5.6) | 21 | -22.4 (-39.3, -5.5) | 22 |
| Week 72 | -20.8 (-41.4, -0.1) | 21 | -25.8 (-45.5 -6.0) | 22 |

### (Example 5: Change in Plasma sVCAM-1 Level)

Table 6-1 and Fig. 5-1 show the average amount of change in the plasmasoluble Vascular Cell Adhesion Molecule-1 (sVCAM) after the assignment. For measurement of the plasma sVCAM level, plasma was prepared from blood collected from each patient, according to a conventional method. The prepared plasma was subjected to a test. For the measurement, a Human sVCAM-1/CD106 Quantikine ELISA Kit (R&D Systems, Inc.) was used.

The placebo group showed almost the same plasma sVCAM values as the value observed at the beginning of the administration, until Week 48 after the beginning of the treatment. However, at Week 72, the value was lower than the value observed at the beginning of the administration.

On the other hand, the MT-3995 group showed lower plasma sVCAM values from Week 24 after the beginning of the administration, and showed the maximum value at Week 72. Based on comparison between the groups, the plasma sVCAM value in the MT-3995 administration group was lower than the value in the placebo group at Week 24, Week 48, and Week 72.

### [Table 6-1]

**Table 6-1. Average amount of change in the plasma sVCAM-1 level after the assignment**

| | Change from baseline / sVCAM-1 (ng/mL) | | | |
|---|---|---|---|---|
| | Placebo | | MT-3995 | |
| Visit | Mean (SD) | (n) | Mean (SD) | (n) |
| Week 12 | 8.3 (136.2) | 23 | 13.8 (121.5) | 24 |
| Week 24 | -3.4 (124.3) | 23 | -43.8 (137.3) | 24 |
| Week 48 | -0.1 (121.8) | 21 | -44.7 (159.3) | 22 |
| Week 72 | -33.7 (147.0) | 21 | -74.7 (182.9) | 22 |

A stratified analysis was carried out for the subjects based on the presence or absence of complication with diabetes. The results are shown in Table 6-2 and Fig. 5-2. DM represents the "group of patients complicated with diabetes". A higher effect was found in the group of patients complicated with diabetes.

### [Table 6-2]

**Table 6-2. Average amount of change in the plasma sVCAM-1 level after the assignment**

| | Change from baseline / sVCAM-1 (ng/mL) | | | | | | | |
|---|---|---|---|---|---|---|---|---|
| | Placebo | | | | MT-3995 | | | |
| | non-DM | | DM | | non-DM | | DM | |
| Visit | Mean | (n) | Mean | (n) | Mean | (n) | Mean | (n) |
| Week 12 | 12.87 | 15 | -0.13 | 8 | 34.25 | 12 | -6.58 | 12 |
| Week 24 | 8.80 | 15 | -26.38 | 8 | -40.58 | 12 | -47.08 | 12 |
| Week 48 | 5.47 | 15 | -14.00 | 8 | -26.64 | 12 | -62.73 | 12 |
| Week 72 | -24.87 | 15 | -55.83 | 8 | -37.18 | 12 | -112.27 | 12 |

Further, a stratified analysis was carried out for the subjects based on the presence or absence of complication with hypertension. The results are shown in Table 6-3 and Fig. 5-3. HP represents the "group of patients complicated with hypertension". A higher effect was found in the group of patients complicated with hypertension.

### [Table 6-3]

**Table 6-3. Average amount of change in the plasma sVCAM-1 level after the assignment**

| | Change from baseline / sVCAM-1 (ng/mL) | | | | | | | |
|---|---|---|---|---|---|---|---|---|
| | Placebo | | | | MT-3995 | | | |
| | non-HP | | HP | | non-HP | | HP | |
| Visit | Mean | (n) | Mean | (n) | Mean | (n) | Mean | (n) |
| Week 12 | 34.55 | 11 | -15.67 | 12 | 30.13 | 8 | 5.69 | 16 |
| Week 24 | 17.09 | 11 | -22.25 | 12 | -43.38 | 8 | -44.06 | 16 |
| Week 48 | -32.82 | 11 | 35.90 | 12 | 0.13 | 8 | -70.29 | 16 |
| Week 72 | -58.91 | 11 | -6.00 | 12 | -48.75 | 8 | -89.57 | 16 |

### (Example 6: Change in Liver Stiffness)

The liver stiffness after the assignment was measured using Shear Wave Elastography or Fibro scan. Table 7 and Fig. 6 show the average amount of change in the liver stiffness according to Shear Wave Elastography. Table 8 and Fig. 7 show the average amount of change in the liver stiffness according to Fibro scan.

According to the measurement by either measurement apparatus, the liver stiffness in the placebo group was found to have been unchanged throughout the treatment period. However, throughout the treatment period, the liver stiffness in the MT-3995 group was lower than the value observed at the beginning of the administration.

### [Table 7]

**Table 7. Average amount of change in the liver stiffness (Shear Wave Elastography) after the assignment**

| | Change from baseline / Liver stiffness (kPa) | | | |
|---|---|---|---|---|
| | Placebo | | MT-3995 | |
| Visit | Mean (SD) | (n) | Mean (SD) | (n) |
| Week 12 | 0.02 (2.61) | 5 | -4.57 (2.48) | 3 |
| Week 24 | 0.64 (5.38) | 5 | -5.63 (3.23) | 3 |
| Week 48 | 0.02 (3.61) | 5 | -4.90 (4.38) | 2 |
| Week 72 | 0.70 (4.61) | 5 | -6.00 (5.23) | 2 |

### [Table 8]

**Table 8. Average amount of change in the liver stiffness (Fibro scan) after the assignment**

| | Change from baseline / Liver stiffness (kPa) | | | |
|---|---|---|---|---|
| | Placebo | | MT-3995 | |
| Visit | Mean (SD) | (n) | Mean (SD) | (n) |
| Week 12 | -0.31 (3.06) | 15 | -0.49 (2.24) | 14 |
| Week 24 | -0.05 (4.01) | 15 | 0.30 (2.82) | 14 |
| Week 48 | 0.87 (3.61) | 12 | -1.05 (3.67) | 13 |
| Week 72 | 0.53 (3.48) | 12 | -1.41 (3.97) | 14 |

## Claims

1. A prophylactic agent or therapeutic agent for human non-alcoholic steatohepatitis, comprising:
the 1,4-benzoxazine compound represented by the following Formula (I): 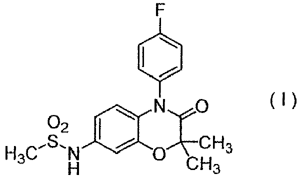 or a pharmaceutically acceptable salt thereof, as an effective component; and
a pharmaceutically acceptable carrier;
the agent being capable of reducing body weight.

2. A prophylactic agent or therapeutic agent for human non-alcoholic steatohepatitis, comprising:
the 1,4-benzoxazine compound represented by the following Formula (I): 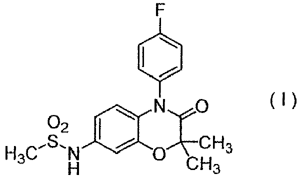 or a pharmaceutically acceptable salt thereof, as an effective component; and
a pharmaceutically acceptable carrier;
the agent being capable of changing the amount of HA, the amount of PIIINP, and the amount of TIMP-1, in serum, and changing the amount of sVCAM-1 in plasma.

3. A prophylactic agent or therapeutic agent for human non-alcoholic steatohepatitis, comprising:
the 1,4-benzoxazine compound represented by the following Formula (I): 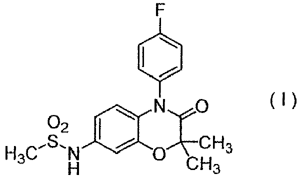 or a pharmaceutically acceptable salt thereof, as an effective component; and
a pharmaceutically acceptable carrier;
the agent being capable of reducing liver stiffness.

4. The prophylactic agent according to any one of claims 1 to 3, capable of suppressing progression from non-alcoholic simple fatty liver to non-alcoholic steatohepatitis.

5. The prophylactic agent or therapeutic agent according to any one of claims 1 to 3, capable of suppressing progression from non-alcoholic steatohepatitis to non-alcoholic liver fibrosis.

6. The prophylactic agent or therapeutic agent according to any one of claims 1 to 3, capable of suppressing progression from non-alcoholic steatohepatitis to liver cancer.

7. A method of prevention or treatment of human non-alcoholic steatohepatitis, comprising:
administering a prophylactically or therapeutically effective amount of the 1,4-benzoxazine compound represented by the following Formula (I): 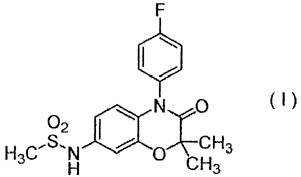 or a pharmaceutically acceptable salt thereof to a patient in need of the prevention or treatment;
the method being capable of reducing body weight.

8. A method of prevention or treatment of human non-alcoholic steatohepatitis, comprising:
administering a prophylactically or therapeutically effective amount of the 1,4-benzoxazine compound represented by the following Formula (I): 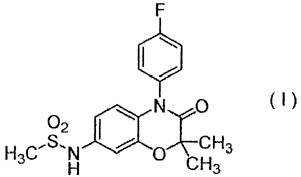 or a pharmaceutically acceptable salt thereof to a patient in need of the prevention or treatment;
the method being capable of changing the amount of HA, the amount of PIIINP, and
the amount of TIMP-1, in serum, and changing the amount of sVCAM-1 in plasma.

9. A method of prevention or treatment of human non-alcoholic steatohepatitis, comprising:
administering a prophylactically or therapeutically effective amount of the 1,4-benzoxazine compound represented by the following Formula (I): 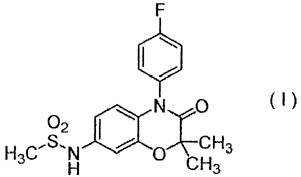 or a pharmaceutically acceptable salt thereof to a patient in need of the prevention or treatment;
the method being capable of reducing liver stiffness.

10. Use of the 1,4-benzoxazine compound represented by the following Formula (I): 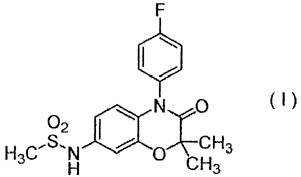 or a pharmaceutically acceptable salt thereof, for the production of a prophylactic agent or therapeutic agent for human non-alcoholic steatohepatitis, the agent being capable of reducing body weight.

11. Use of the 1,4-benzoxazine compound represented by the following Formula (I): 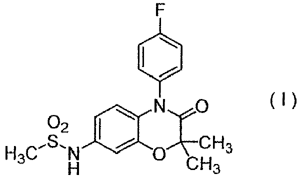 or a pharmaceutically acceptable salt thereof, for the production of a prophylactic agent or therapeutic agent for human non-alcoholic steatohepatitis, the agent being capable of changing the amount of HA, the amount of PIIINP, and the amount of TIMP-1, in serum, and changing the amount of sVCAM-1 in plasma.

12. Use of the 1,4-benzoxazine compound represented by the following Formula (I): 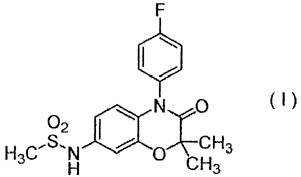 or a pharmaceutically acceptable salt thereof, for the production of a prophylactic agent or therapeutic agent for human non-alcoholic steatohepatitis, the agent being capable of reducing liver stiffness.
